# EUROPEAN PATENT SPECIFICATION

(11) **EP 0 441 348 B1**
(45) Date of publication and mention of the grant of the patent: **20.04.1994**
(21) Application number: 91101615.2
(22) Date of filing: 06.02.1991
(51) Int. Cl.: B01D 45/14, G01N 33/00

(54) **Gas-liquid separator**
Gas-Flüssigkeitsabscheider
Séparateur gaz-liquide

(30) Priority: 07.02.1990 JP 27884/90
(43) Date of publication of application: 14.08.1991
(73) Proprietor: HORIBA, LTD., Minami-ku Kyoto (JP)
(72) Inventor: Koike, Hideki, Momoyama Fusimi-ku, Kyoto (JP); Aoki, Junji, Okazaki Sakyo-ku, Kyoto (JP)
(74) Representative: TER MEER STEINMEISTER & PARTNER GbR

(56) References cited:
- US-A- 4 678 488

## Description

### Background of the Invention

### Field of the Invention

The present invention relates to a gas-liquid separator as claimed in the preamble of claim 1.

### Description of the Prior Art

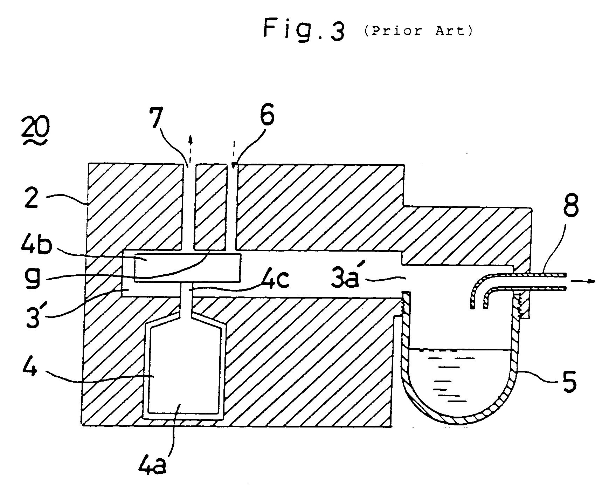

Various kinds of gas-liquid separators for separating a gas and a liquid from each other containing impurities in a centrifugal separator and the like have been proposed and have been used also as an assistant device of an infrared analyzer for measuring an exhaust gas of a motor vehicle to analyse air pollutants. Usually, CO₂, NOₓ, HC and the like are contained in said exhaust gas of a motor vehicle. In order to analyze these air pollutants, the infrared analyzer, in which ingredients within said gas are analyzed by the use of infrared rays, has been advantageously used. However, in case where the exhaust gas is analyzed in this infrared analyzer, if moisture and solid impurities are contained in the sample gas, errors occur in results of analysis, so that a gas-liquid separator having a high performance has been desired. So, a gas-liquid separator shown in Fig. 3 has been adopted.

This gas-liquid separator 20 is disposed between an engine (not shown) and said infrared analyzer and a body portion 2 is provided with a gas-liquid separating chamber 3', a centrifugal separator 4 and a drain pot 5.

Said gas-liquid separating chamber 3' is a nearly rectangular hollow chamber and a sample gas-introducing pipe 6 connected with an exhaust pipe of said engine at one end thereof and a sample gas-discharging pipe 7 connected with a vacuum suction pump at one end thereof are arranged above the gas-liquid separating chamber 3'.

Said centrifugal separator 4 comprises a motor 4a and a rotating plate 4b. Said motor 4a is arranged below the gas-liquid separating chamber 3' and an axis shaft line of the motor 4a coincides with an extending direction of said sample gas-discharging pipe 7. A small gap (g) is formed between said rotating plate 4b facing to openings of said sample gas-introducing pipe 6 and the sample gas-discharging pipe 7 and an upper surface of the gas-liquid separating chamber 3' so that said sample gas flowing into the gas-liquid separating chamber 3' through the sample gas-introducing pipe 6 may be efficiently flown out to the side of the sample gas-discharging pipe 7 without being retained within the gas-liquid separating chamber 3'.

Said drain pot 5 is a detachable liquid-storing vessel made of glass and/or transparent plastics and installed by screwing in a screw member formed in an exhaust port 3a' of the gas-liquid separating chamber 3'. An end portion of a by-pass pipe 8 communicated with a vacuum suction pump for use in a by-pass (not shown) is extended above the drain pot 5 so that the gas-liquid separating chamber 3' may be kept at a negative pressure during the time when said vacuum suction pump for use in a by-pass is operated to introduce the separated liquid into the drain pot 5. In addition, the sample gas-discharging pipe 7 is provided with a pressure regulator so that a gas pressure of the sample gas supplied to the infrared analyzer can be regulated when a gas pressure of the sample gas introduced into the sample gas-discharging pipe 7 is changed.

In case where an exhaust gas is measured in said gas-liquid separator 20, both the vacuum suction pump for use in sampling and the vacuum suction pump for use in a by-pass are started to keep the gas-liquid separating chamber 3' at a negative pressure of for example about -2,94 N/cm² (-0,3 Kgf/cm²). Simultaneously, the centrifugal separator 4 is operated. Thereupon, a part of the exhaust gas discharged from said engine is introduced into the gas-liquid separating chamber 3' as the sample gas through the sample gas-introducing pipe 6. At this time, liquids, such as water drops, and small particulate solids, such as soot, come into collision with the rotating plate 4b rotating with high speed to be blown off by a centrifugal separation. Thus, upon separating the sample gas into liquids and gases, the sample gas containing no liquid and the like is introduced into the infrared analyzer from said small gap (g) through the sample gas-discharging pipe 7. And, the ingredients of the sample gas are analyzed in this infrared analyzer.

On the other hand, said liquids and the like separated are released into the gas-liquid separating chamber but the gas-liquid separating chamber 3' is kept at a negative pressure, so that moisture, soot and the like, which are heavier than the gas can fall into the drain pot 5 to be stored therein.

However, a quantity of the liquids and the like stored in the drain pot 5 is increased with a continued operation of the engine. If a considerable quantity of liquids and the like was stored within the drain pot 5, the drain pot 5 is suitably removed and the liquids and the like are dumped followed by installing the drain pot 5 again. Thus, the sample gas can be analyzed.

However, with the gas-liquid separator 20 of this kind, the liquids and the like must be dumped at every time when they are accumulated in the drain pot 5. The attachment and detachment of the drain pot 5 is troublesome and its timely check is necessary, so that the maintenance is troublesome.

In addition, the gas-liquid separating chamber 3' is adapted to be kept at the negative pressure and connected with said vacuum suction pump for use in a by-pass through a by-pass pipe 8. However, the suction pressure of the vacuum suction pump for use in a by-pass is not able to follow the internal pressure of the gas-liquid separating chamber 3' changing with a fluctuation of an output of the engine. As a result, in case the output of the engine is increased and said internal pressure of the gas-liquid separating chamber 3' is likewise increased, the negative pressure condition is broken and thus it is not possible to suck the centrifugally separated liquid fractions toward the side of the drain pot 5. The centrifugally separated liquid fractions do not fall into the drain pot 5 but a part thereof is flown into the side of the by-pass pipe 8 according to circumstances, Therefore, soot and the like is stucked to the by-pass pipe 8 to choke it in an extreme case.

### Summary of the invention

The present invention has been achieved in view of the above described problems and it is an object of the present invention to provide a gas-liquid separator capable of surely separating a gas from a liquid even though a pressure of a gas flowing into a gas-liquid separating chamber is fluctuated and requiring no troublesome maintenance and the like.

In order to achieve the above described object, a gas-liquid separator comprising a gas-liquid separating chamber provided with a gas-introducing pipe, a gas-discharging pipe and a by-pass pipe connected therewith, and a centrifugal separator for centrifugally separating a gas flown into said gas-liquid separating chamber through said gas-introducing pipe into a gas flown out toward the side of said gas-discharging pipe and a liquid, and in which liquid fractions centrifugally separated in said centrifugal separator are discharged toward the side of said by-pass pipe. The gas-liquid separator is characterized in that said gas-liquid separating chamber is provided with a communicating pipe communicated therewith and said communicating pipe is provided with a pressure regulator for regulating an internal pressure of the gas-liquid separating chamber to an appointed pressure depending upon a fluctuation of a gas pressure on the side of the gas-introducing pipe therein.

With the above described construction, the gas flown into the gas-liquid separating chamber through the gas-introducing pipe is separated into said gas and said liquid by means of the centrifugal separator. And, the gas is flown into the side of the gas-discharging pipe to be discharged while the liquid is discharged to the side of the by-pass pipe.

In this time, when a pressure of said gas flown into the gas-liquid separating chamber is fluctuated, also said internal pressure of the gas-liquid separating chamber begins to be changed but said gas pressure within the gas-liquid separating chamber is regulated by an operation of said pressure regulator and thus the internal pressure can be kept nearly constant, whereby not only the gas-liquid separation can be appropriately conducted but also the liquid can be discharged without being retained within the by-pass pipe and the like.

### Brief Description of the Drawings

Fig. 1 is a sectional view showing a gas-liquid separator according to one preferred embodiment of the present invention;
Fig. 2 is a sectional view showing a gas-liquid separator according to another preferred embodiment of the present invention; and
Fig. 3 is a sectional view showing the conventional gas-liquid separator.

### Description of the Preferred Embodiment

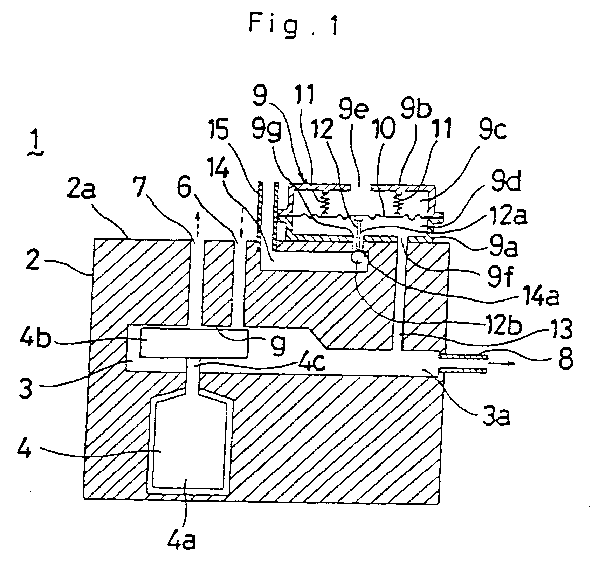

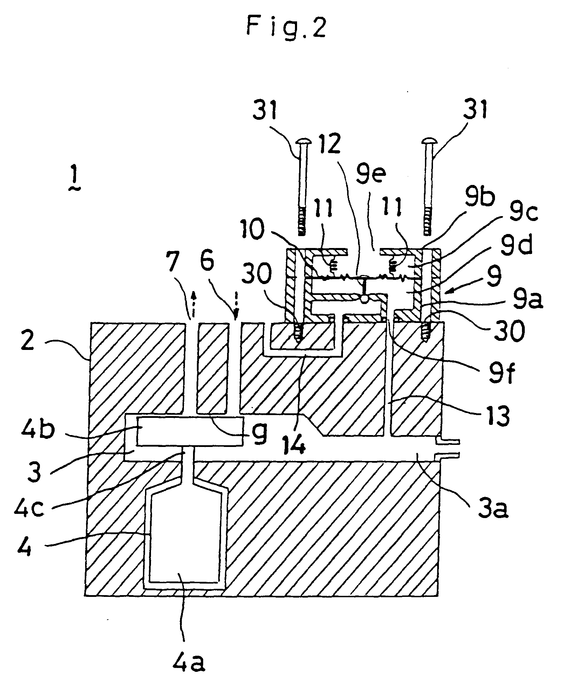

Preferred embodiments of the present invention are below described with reference to the drawings. In addition, in Figs. 1 and 2 members corresponding to those in Fig. 3 are designated by same reference numerals as in Fig. 3 in order to omit the repeated description.

A gas-liquid separator 1 according to Fig. 1 comprises a body portion 2 provided with a pressure regulator 9. Further, a pipe end of a by-pass pipe 8 communicating with a vacuum suction pump for use in a by-pass is connected with a discharge port 3a of a gas-liquid separating chamber 3.

Said pressure regulator 9 comprises an upper chamber 9c and a lower chamber 9d formed by disposing a diaphragm 10 at a portion where a case body 9a is spliced to a cover member 9b and is fixedly mounted on an upper surface 2a of said body portion 2. Two pieces of spring members 11, 11 are disposed between an upper surface of said diaphragm 10 and an inner surface of said cover member 9b. An upper end of a valve member 12 having a spherical member 12b fixed on a short axis 12a is fixedly mounted with said short axis 12a on a center of a lower surface of the diaphragm 10. In addition, the cover member 9b is provided with an opening 9e communicating with an air and also said case body 9a is provided with two openings 9f, 9g so that one opening 9f may communicate with said gas-liquid separating chamber 3 through a communicating pipe 13. Furthermore, the other opening 9g communicates with an opening 14a of a hollow portion 14 formed in an upper portion of the body portion 2, said short axis 12a of said valve member 12 being inserted into said opening 14a and said opening 9g, and said spherical member 12b being arranged within said hollow portion 14.

An air-supply pipe 15 provided with an air-release means or an air filter is connected with the hollow portion 14 at an end portion thereof to supply the pressure regulator 9 with a pressure-regulating gas from an air through the hollow portion 14.

The pressure regulator 9 is adapted to regulate a quantity of said pressure-regulating gas supplied depending upon the pressure fluctuation of the gas exhausted at a start of the engine. A force of said spring members are regulated so that the diaphragm 10 may be horizontally extended to form a small gap between the opening 14a of the hollow portion 14 and the spherical member 12b at a stationary pressure.

Next, an operation is described.

In the measurement, a vacuum suction pump for use in sampling and said vacuum suction pump for use in a by-pass are started to keep an inside of the gas-liquid separating chamber 3 at a negative pressure of about -2,94 N/cm² (-0,3 Kgf/cm²). Thus, the pressure regulator 9 is supplied with the pressure-regulating gas from an air through said air-supply pipe 15 and the hollow portion 14.

In addition, a centrifugal separator 4 is started. Further, a part of an exhaust gas exhausted from the engine is introduced into the gas-liquid separator 3 as a sample gas through a sample gas-introducing pipe 6. At this time, liquids, such as water drops, and small particulate solids, such as soot, contained in said sample gas come into collision with a rotary member 4b rotating with high speed to be blown off by a centrifugal separation. When the sample gas is separated into liquids and gases in the above described manner, the sample gas containing no liquid is sucked by said vacuum suction pump for use as a sample to be introduced into an infrared analyzer from said small gap (g) through a sample gas-discharging pipe 7. And, ingredients are analyzed in said infrared analyzer.

On the other hand, said liquids and the like are sucked from the gas-liquid separating chamber 3 kept at the negative pressure to be discharged to the side of said by-pass pipe 8. At this time, since the pressure-regulating gas supplied to the pressure regulator 9 is flown out to the side of the by-pass pipe 8 through said communicating pipe 13 and an exhaust port 3a of the gas-liquid separating chamber 3, the liquids stained with moisture, soot and the like are exhausted without sticking to said exhaust port 3a and an inside of the by-pass pipe 8.

However, the pressure at a sample inlet communicating with the gas-introducing pipe 6 is varied within a range of about -0,98 to 9,8 N/cm² (-0,1 to 1 Kgf/cm²) due to the fluctuation of the output of the engine and thus also the internal pressure of the gas-liquid separating chamber 3 begins to be varied but a pressure in the vicinity of said exhaust port 3a, which is a pressure-regulating point of the gas-liquid separating chamber 3, is regulated to keep the inside of the gas-liquid separating chamber 3 at a constant pressure. For example, if the desired regulated pressure is P, the atmospheric pressure being p₀, the area of the diaphragm is A, and the force of the springs being kx, the following force balance relationship is provided:$\text{PA=p₀A-kx}$

Now, if the output of the engine is increased to increase the internal pressure of the gas-liquid separating chamber 3, the diaphragm 10 of the pressure regulator 9 is bent upward to move up the valve member 12. At this time, the diaphragm 10 is deformed depending upon the fluctuation of pressure to reduce an openness between the spherical member 12b of the valve member 12 and the opening 14a, whereby reducing a quantity of the pressure-regulating gas flown into the pressure-regulator 9. At this time, the quantity of the pressure-regulating gas flown into the exhaust port 3a through said communicating pipe 13 from the pressure regulator 9 is reduced with an increase of the sample gas flown into the gas-liquid separating chamber 3.

Accordingly, even though the internal pressure of the gas-liquid separating chamber 3 begins to be varied with the fluctuation of the output of the engine, the total flow rate of the gas passing over said pressure-regulating point is unchanged, so that said pressure at the pressure-regulating point is kept constant equally to that before the output is increased. Thus, not only the liquids centrifugally separated can be fluently discharged into the by-pass pipe 8 through the exhaust port 3a but also the gases supplied into said sample gas-discharging pipe 7 through the small gap (g) and to the infrared analyzer as the sample gas are regulated in pressure. In addition, when the output of the engine arrives at the maximum value, the internal pressure of the pressure-regulator 9 is increased to cut off the supply of the pressure-regulating gas by means of the valve member 12, whereby a pressure-regulating capacity is lost. However, the force of the springs, the suction force of the vacuum pumps and the like can previously be regulated so that said supply of the pressure-regulating gas may not be cut off. In short, the pressure-regulator is adapted to always satisfactorily operate. On the other hand, when the pressure within the gas-liquid separating chamber 3 begins to be reduced with a reduction of the output of the engine, the valve member 12 is opened to increase the quantity of the pressure-regulating gas supplied. Accordingly, the flow rate at the pressure-regulating point can be always kept nearly unchanged and thus the discharge of the liquids separated and the supply of the sample gas to the infrared analyzer are not influenced.

In addition, although the gas-liquid separator 1 is disposed between the engine and the infrared analyzer in order to analyze an exhaust gas from a motor vehicle in the above described preferred embodiment, the present invention is not limited by this. That is the present invention can be widely applied to various kinds of apparatus for centrifugally separating a liquid-containing gas.

Furthermore, the pressure regulator 9 may be detachably mounted on the body portion 2, as shown in Fig. 2. Besides, referring to Fig. 2, reference numeral 30 designates screw holes and reference numeral 31 designates screws.

As above described, the gas-liquid separator according to the present invention is provided with a pressure regulator communicating with the gas-liquid separating chamber, so that the internal pressure of the gas-liquid separating chamber can be kept nearly unchanged even though the gas pressure on the side of the gas introducing pipe is fluctuated. Accordingly, the centrifugal separation of the gas can be appropriately achieved and thus the liquids and the like can be discharged without being retained in the by-pass pipe and the like, whereby the problems of the choking and staining can be prevented. In addition, the troublesome maintenance, such as the removal of the drain pot followed by the dumping of the liquids stored in the drain pot after every appointed time, becomes unnecessary.

## Claims

1. A gas-liquid separator (1) comprising a gas-liquid separating chamber (3) provided with a gas-introducing pipe (6), a gas-discharging pipe (7) and a by-pass pipe (8) connected therewith, and with a centrifugal separator (4a-4c) for centrifugally separating a gas flown into said gas-liquid separating chamber (3) through said gas-introducing pipe (6) into a gas flown out toward the side of said gas-discharging pipe (7) and a liquid, and in which liquid fractions centrifugally separated by said centrifugal separator (4a-4c) are discharged toward the side of said by-pass pipe (8), **characterized in that**
- said gas-liquid separating chamber (3) is provided with a communicating pipe (13) communicated therewith and
- said communicating pipe (13) is provided with a pressure regulator (9) for regulating an internal pressure of the gas-liquid separating chamber (3) to an appointed pressure depending upon a fluctuation of a gas pressure on the side of the gas-introducing pipe (6).

2. The gas-liquid separator as claimed in claim 1, **characterized in that** the pressure regulator (9) is divided by a diaphragm (10) into a first chamber (9c) receiving a constant pressure and into a second chamber (9d) communicated with said communicating pipe (13) and via a further opening (9g) with a hollow portion (14) containing a valve member (12) connected to said diaphragm (10) in order to open or close said opening (9g) in dependence of a control gas pressure in said hollow portion (14).

3. The gas-liquid separator as claimed in claim 2, **characterized in that** the fluctuation of the control gas pressure corresponds to the fluctuation of the gas pressure on the side of the gas-introducing pipe (6).

4. The gas-liquid separator as claimed in claims 2 or 3, **characterized in that** load means (11) are provided for loading the diaphragm (10) with a predetermined force.

5. The gas-liquid separator as claimed in one of the claims 2 to 4, **characterized** **in that** the hollow portion (14) is provided in a supporting member (2) to which the pressure regulator (9) is connected.

6. The gas-liquid separator as claimed in one of the claims 2 to 4, **characterized** **in that** the hollow portion (14) is built up by a third chamber of the pressure regulator (9).

7. The gas-liquid separator as claimed in claim 6, **characterized in that** the pressure regulator (9) is detachably mounted on a supporting member (2).

## Patentansprüche

1. Gas-Flüssigkeits-Trennvorrichtung (1), mit einer Gas-Flüssigkeits-Trennkammer (3), die mit einer Gas-Einströmleitung (6), einer Gas-Ausströmleitung (7) und einem Umgehungsrohr (8) verbunden ist, und mit einer Zentrifugaltrennvorrichtung (4a bis 4c) zum zentrifugalen Trennen eines in die Gas-Flüssigkeits-Trennkammer (3) durch die Gas-Einströmleitung (6) einströmenden Gases in ein zur Seite der Gas-Ausströmleitung (7) strömendes Gas sowie in eine Flüssigkeit, die durch die Zentrifugaltrennvorrichtung (4a bis 4c) zur Seite des Umgehungsrohrs (8) ausgestoßen wird, **dadurch gekennzeichnet,** daß
- die Gas-Flüssigkeits-Trennkammer (3) mit einer kommunizierenden Leitung (13) verbunden ist, und
- die kommunizierende Leitung (13) mit einem Druckregler (9) zum Regulieren eines Innendrucks der Gas-Flüssigkeits-Trennkammer (3) auf einen festgelegten Druckje nach Fluktuation des Gasdrucks auf der Seite der Gas-Einströmleitung (6) ausgestattet ist.

2. Gas-Flüssigkeits-Trennvorrichtung nach Anspruch 1, **dadurch gekenn****zeichnet,** daß der Druckregler (9) durch ein Diaphragma (10) in eine erste Kammer (9c), in der ein konstanter Druck herrscht sowie in eine mit der kommunizierenden Leitung (13) verbundene zweite Kammer (9d) unterteilt ist, welche über eine weitere Öffnung (9g) mit einem Hohlraum (14) in Verbindung steht, der ein mit dem Diaphragma (10) verbundenes Ventilelement (12) enthält, um die Öffnung (9g) in Abhängigkeit eines Steuergasdrucks im Hohlraum (14) zu öffnen oder zu schließen.

3. Gas-Flüssigkeits-Trennvorrichtung nach Anspruch 2, **dadurch ge****kennzeichnet,** daß die Fluktuation des Steuergasdrucks der Fluktuation des Gasdrucks an der Seite der Gas-Einströmleitung (6) entspricht.

4. Gas-Flüssigkeits-Trennvorrichtung nach einem der Ansprüche 2 oder 3, **dadurch gekennzeichnet,** daß Beaufschlagungseinrichtungen (11) zum Beaufschlagen des Diaphragmas (10) mit einer vorbestimmten Kraft vorhanden sind.

5. Gas-Flüssigkeits-Trennvorrichtung nach einem der Ansprüche 2 bis 4, **dadurch gekennzeichnet,** daß der Hohlraum (14) in einem Trägerelement (2) vorhanden ist, mit dem der Druckregler (9) verbunden ist.

6. Gas-Flüssigkeits-Trennvorrichtung nach einem der Ansprüche 2 bis 4, **dadurch gekennzeichnet,** daß der Hohlraum (14) durch eine dritte Kammer des Druckreglers (9) gebildet ist.

7. Gas-Flüssigkeits-Trennvorrichtung nach Anspruch 6, **dadurch ge**kennzeichnet, daß der Druckregler (9) loslösbar am Trägerelement (2) montiert ist.

## Revendications

1. Un séparateur gaz-liquide (1) comprenant une chambre de séparation gaz-liquide (3) pourvue d'un conduit d'introduction de gaz (6), d'un conduit d'évacuation de gaz (7) et d'un conduit de by-pass (8) relié à ceux-ci, ainsi qu'un séparateur centrifuge (4a-4c) pour séparer de manière centrifuge un gaz pénétrant dans ladite chambre de séparation gaz-liquide (3) par ledit conduit d'introduction de gaz (6) de façon à former un gaz s'échappant par ledit conduit d'évacuation de gaz (7) et un liquide, et dans lequel les fractions liquides séparées de façon centrifuge par ledit séparateur centrifuge (4a-4c) sont évacuées par ledit conduit de by-pass (8), ce séparateur étant caractérisé en ce que la chambre de séparation liquide-gaz (3) est munie d'un conduit de communication (13) qui établit une communication avec cette chambre et en ce que ledit conduit de communication (13) est pourvu d'un régulateur de pression (9) pour régler la pression interne de la chambre de séparation liquide-gaz (3) à une pression déterminée, fonction de la fluctuation de la pression du gaz règnant dans le conduit d'introduction de gaz (6).

2. Le séparateur gaz-liquide selon la revendication 1, caractérisé en ce que le régulateur de pression (9) est divisé par un diaphragme (10) et une première chambre (9c) soumise à une pression constante, et en une deuxième chambre (9d) communiquant avec ledit conduit de communication (13) et, par l'intermédiaire d'une autre ouverture (9g) avec une partie creuse (14) contenant une vanne (12) reliée au diaphragme (10) afin d'ouvrir ou de fermer ladite ouverture (9g) en fonction de la pression du gaz de contrôle dans ladite portion creuse (14).

3. Le séparateur gaz-liquide selon la revendication 2, caractérisé en ce que la fluctuation de la pression du gaz de contrôle correspond à la fluctuation de la pression du gaz règnant dans le conduit d'introduction du gaz (6).

4. Le séparateur gaz-liquide ainsi que revendiqué dans les revendications 2 ou 3, caractérisé en ce que des éléments de charge (11) sont prévus pour exercer sur le diaphragme (10) une force prédéterminée.

5. Le séparateur gaz-liquide ainsi que revendiqué dans l'une des revendications 2 à 4, caractérisé en ce que la partie creuse (14) est installée dans un élément support (2) auquel le régulateur de pression (9) est relié.

6. Le séparateur gaz-liquide ainsi que revendiqué dans l'une des revendications 2 à 4, caractérisé en ce que la partie creuse (14) est formée par une troisième chambre du régulateur de pression (9).

7. Le séparateur gaz-liquide ainsi que revendiqué dans la revendication 6, caractérisé en ce que le régulateur de pression (9) est monté de façon détachable sur un élément support (2).
